# EUROPEAN PATENT SPECIFICATION

(11) **EP 1 363 654 B1**
(45) Date of publication and mention of the grant of the patent: **30.07.2008**
(21) Application number: 01986546.8
(22) Date of filing: 20.12.2001
(51) Int. Cl.: A61K 31/09, A61K 38/44, C12N 9/02, G01N 33/50, C12N 15/10, A61P 33/06

(54) **FAB I AND INHIBITION OF APICOMPLEXAN PARASITES**
FAB I UND HEMMUNG VON APICOMPLEXAN-PARASITEN
GENE FAB I ET L'INHIBITION DES PARASITES APICOMPLEXANS

(30) Priority: 21.12.2000 US 257771 P; 26.01.2001 US 264499 P
(43) Date of publication of application: 26.11.2003
(73) Proprietor: Mcleod, Rima W., Chicago, IL 60637 (US); Muench, Stephen P., Stockport, Manchester SK1 4HQ (GB); Rafferty, John B., Crookes, Sheffield S10 1SE (GB); Kyle, Dennis E., APO AP 96553 (US); Mui, Ernest, Chicago, IL 60616 (US); Kirisits, Michael, Chicago, IL 60657 (US); Mack, Douglas G., Riverside, IL 60546 (US); Roberts, Craig W., Glasgow, G12 0TW (GB); Samuel, Benjamin, Chicago, IL 60657 (US); Lyons, Russel E., Jindalee, QLD 4074 (AU); Milhous, Wilbur K., Germantown, MD 20874 (US); Rice, David W., Sheffield S11 9SN (GB); Prigge, Sean, Fitzwilliam, NH 03447 (US)
(72) Inventor: Mcleod, Rima W., Chicago, IL 60637 (US); Muench, Stephen P., Stockport, Manchester SK1 4HQ (GB); Rafferty, John B., Crookes, Sheffield S10 1SE (GB); Kyle, Dennis E., APO AP 96553 (US); Mui, Ernest, Chicago, IL 60616 (US); Kirisits, Michael, Chicago, IL 60657 (US); Mack, Douglas G., Riverside, IL 60546 (US); Roberts, Craig W., Glasgow, G12 0TW (GB); Samuel, Benjamin, Chicago, IL 60657 (US); Lyons, Russel E., Jindalee, QLD 4074 (AU); Milhous, Wilbur K., Germantown, MD 20874 (US); Rice, David W., Sheffield S11 9SN (GB); Prigge, Sean, Fitzwilliam, NH 03447 (US)
(74) Representative: Grund, Martin
(86) International application number: PCT/US2001/049738
(87) International publication number: WO 2002/049576

(56) References cited:
- WO-A-00/72846
- WO-A-01/00138
- US-A- 6 071 518
- MCLEOD R ET AL: "Triclosan inhibits the growth of Plasmodium falciparum and Toxoplasma gondii by inhibition of apicomplexan Fab I." INTERNATIONAL JOURNAL FOR PARASITOLOGY. FEB 2001, vol. 31, no. 2, February 2001 (2001-02), pages 109-113, XP002322349 ISSN: 0020-7519
- SUROLIA N ET AL: "Triclosan offers protection against blood stages of malaria by inhibiting enoyl-ACP reductase of Plasmodium falciparum." NATURE MEDICINE. FEB 2001, vol. 7, no. 2, February 2001 (2001-02), pages 167-173, XP001205625 ISSN: 1078-8956
- SUGUNA K ET AL: "Structural basis for triclosan and NAD binding to enoyl-ACP reductase of Plasmodium falciparum." BIOCHEMICAL AND BIOPHYSICAL RESEARCH COMMUNICATIONS. 27 APR 2001, vol. 283, no. 1, 27 April 2001 (2001-04-27), pages 224-228, XP002322350 ISSN: 0006-291X
- LEVY C W ET AL: "Molecular basis of triclosan activity." NATURE. 1 APR 1999, vol. 398, no. 6726, 1 April 1999 (1999-04-01), pages 383-384, XP001205986 ISSN: 0028-0836
- HOANG TUNG T ET AL: "Characterization of Pseudomonas aeruginosa enoyl-acyl carrier protein reductase (FabI): A target for the antimicrobial triclosan and its role in acylated homoserine lactone synthesis" JOURNAL OF BACTERIOLOGY, WASHINGTON, DC, US, vol. 181, no. 17, 1999, pages 5489-5497, XP002194160 ISSN: 0021-9193
- ROUJEINIKOVA A ET AL: "Crystallographic analysis of triclosan bound to enoyl reductase" JOURNAL OF MOLECULAR BIOLOGY, LONDON, GB, vol. 294, no. 2, 26 November 1999 (1999-11-26), pages 527-535, XP004464272 ISSN: 0022-2836
- WALLER R F ET AL: "NUCLEAR-ENCODED PROTEINS TARGET TO THE PLASTID IN TOXOPLASMA GONDIIAND PLASMODIUM FALCIPARUM" PROCEEDINGS OF THE NATIONAL ACADEMY OF SCIENCES OF THE UNITED STATES, NATIONAL ACADEMY OF SCIENCE, WASHINGTON, DC, US, vol. 95, no. 21, 13 October 1998 (1998-10-13), pages 12352-12357, XP001036851 ISSN: 0027-8424

## Description

### BACKGROUND

Discovery and characterization of an apicomplexan Fab I gene and encoded enzyme and the discovery of triclosan as a lead compound, provide means to rationally design novel inhibitory compositions useful for prevention and treatment of apicomplexan and microbial related diseases.

Fab I, enoyl acyl carrier protein reductase (ENR), is an enzyme used in fatty acid synthesis. It is a single chain polypeptide in plants, bacteria, and mycobacteria, but is part of a complex polypeptide in animals and fungi. Certain other enzymes in fatty acid synthesis in apicomplexan parasites appear to have multiple forms, homologous to either a plastid sequence, a plant-like single chain enzyme, or more like the animal complex polypeptide.

Apicomplexan infections are among the most common and devastating infectious diseases. Malaria (Plasmodium) kills one child every eleven seconds and three million people every year. It is a cause of substantial morbidity in pregnant women and young children. Toxoplasmosis gondii results in a chronic central nervous system infection in more than a third of the world population, as well as acute life threatening disease in immunocomprised individuals. New medicines are greatly needed for the treatment of these diseases.

Recently a number of plant-like biochemical pathways associated with the vestigial plastid organelle of *T. gondii* and Plasmodium species have been suggested as new targets for such medicines (Roberts et al, 1998; Waller et al., 1998; Zuther et al., 1999). A particularly attractive target in this respect is the fatty acid biosynthesis pathway because there are major differences between the structure of the plastid-associated enzymes found in plants and the cytosolic enzymes found in mammals (Roberts et al., 1998; Jomaa et al., 1999; Zuther et al., 1999; Waller et al., 2000). Importantly, enzymes of mammalian lipid synthesis form domains on a multifunctional protein, whereas those enzymes in plants and certain bacteria are found on discrete mono-functional polypeptides. These differences have already been exploited by a number of compounds which selectively inhibit bacterial or plant enzymes, but do not inhibit mammalian enzymes (Roberts et al., 1998; Waller et al., 1998; Zuther et al., 1999; Payne et al., 2000). Notably, both *T. gondii* and *P. falciparum* have been shown to possess mono-functional, plant- or bacterial-like fatty acid biosynthesis enzymes which are targeted to the plastid organelle via a bipartite, N-terminal transit sequence (Waller et al., 1998; Zuther et al., 1999; Roos et al., 1999; DeRocher et al., 2000). Compounds such as aryloxyphenoxypropionates (Zuther et al., 1999), cyclohexanedione (Zuther et al., 1999) herbicides and thiolactomycin (Waller et al., 1998) which inhibit acetyl-CoA carbozylase (ACC) and β-ketoacyl-ACP synthase (Fab H) respectively, have been demonstrated to restrict the growth of *T. gondii in vitro*.

WO01 /00138 is state of the art according to Article 54 (3) EPC and discloses the use of Triclosan as an antimalarial and identifies FabI as the enzyme in malaria targeted by Triclosan. D4 does not disclose SEQ ID No. 5 and does not mention the use of Triclosan for treating T.gondii infection.

Hoang Tung T. et al. suggest FabI as a target for Triclosan and its use as antimicrobial.

Enoyl acyl carrier protein reductase catalyses the NAD (P)-dependent reduction of a trans-2,3 enoyl moiety into a saturated acyl chain, the second reductive step in the fatty acid biosynthesis pathway. Recent studies on the inhibition of ENR by compounds such as the diazaborines (Turnowsky et al., 1989; Baldock et al., 1996) and triclosan (McMurray et al., 1998; Heath et al., 1998; Levy et al., 1999; Payne et al., 2000; and Jones et al., 2000) have validated this enzyme as a target for the development of new antibacterial agents. In particular, triclosan, which is found in many house-hold formulation including soaps, deodorants, hand lotion, toothpaste and impregnated into plastics as an anti-bacterial agent is an extremely potent ENR inhibitor (Ward et al., 1999). A question is whether Fab I is in apicomplexan parasites and, if so, whether inhibition of Fab I can inhibit parasite growth and/or survival.

### SUMMARY OF THE INVENTION

The present invention relates the first report of apicomplexan Fab I (enoyl acyl carrier protein reductase, ENR) and discloses the effects of triclosan, a potent and specific inhibitor of this enzyme, on the *in vitro* growth of *T. gondii* and *P. falciparum* chain. A plant-like Fab I in *P. falciparum* was identified by the inventors and the structure was modeled on the Brassia napus and Escherichia coli structures, alone and complexed to triclosan (5-chloro-2-[2,4 dichloropheoxyl] phenol), which confirmed all the requisite features of an enoyl acyl carrier protein reductase (ENR) and its interactions with triclosan. Like the remarkable effect of triclosan on a wide variety of bacteria, this compound markedly inhibits growth and survival of the apicomplexan parasites *P. falciparum* and Toxoplasma gondii at low concentrations (i.e., IC50 ≅150-2000 and 62 nanogram/ml respectively).

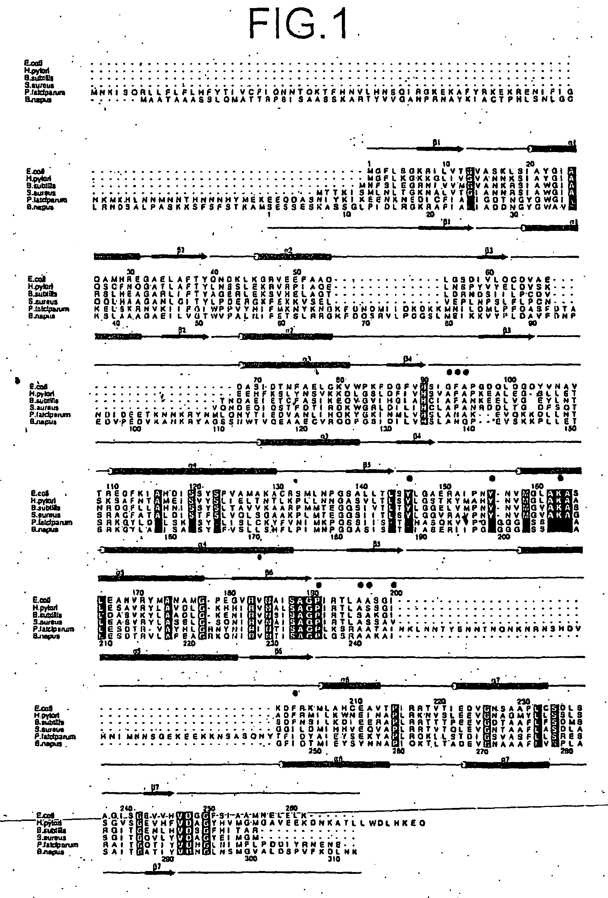

Initially, a sequence for a putative Plasmodium falciparum Fab I was located on the aggregate *P. falciparum* chromosomes referred to as "blob" (GenBank Accession Number AF338731). The deduced amino acid sequence and a multisequence alignment with representative enoyl acyl carrier protein reductases are shown in FIG. 1 (GenBank Accession Number AF33781). GenBank web site is www.ncbi.nlm.nih.gov. The gene sequence of Plasmodium ENR was obtained with a BLAST search using the sequences from both the B. napus and E. coli enzymes within the P. falciparum database "PlasmoDB" (found at www.PlasmoDB.org). (See Materials and Methods). This sequence was then converted to an amino acid sequence at www.expasy.ch/tools/dna.html. The sequence was aligned using the "Multiple Sequence Alignment at http://searchlauncher.bcm.tmc.edu.

Subsequently, the molecules were prepared and tested in a laboratory setting (see Example 1). Errors in the published sequence for the Plasmodium genome were found. FIG. 1 shows the correct amino acid sequence for Plasmodium.

Analysis of the pattern of sequence conservation confirmed that this protein has all the residues that have been identified as essential for enzyme activity. Interestingly, there is much greater sequence similarity with the plant enzyme than with the ENRs of bacterial origin. The *P. falciparum* enoyl acyl carrier protein reductase appears to have a plastid targeting sequence (Waller et al., 2000) and has a number of internal insertions. In addition, the *P. falciparum* protein has an extremely polar additional internal insertion for which no counterpart exists in any of the previously described enoyl acyl carrier protein reductases. This is important to target sequences among that are unique species of Fab I targets that can be attacked with antisense.

Because Fab I was located, the effects of triclosan on Plasmodium falciparum *in vitro* were investigated. For *P. falciparum,* the *in vitro* assays (Milhous et al., 1985; Oduola et al., 1988) were conducted using a modification of the semiautomated microdilution technique for assessing antifolate antagonists. Instead of dialysed human plasma, 10% Albumax I (Gibco BRL), a serum-free substitute, was used to supplement the RPMI 1640 medium. All test compounds were dissolved in DMSO and diluted 400-fold into complete medium before serial dilution over 11 concentrations. Incubation was at 37°C in 5% 02, 5% CO2 and 90% N2 for 48 h. [3H]-Hypoxanthine incorporation was measured as described previously (Milhous et al, 1985; Oduola et al, 1988). *P. falciparum* strain W2 is susceptible to mefloquine, but resistant to pyrimethamine, sulphadoxine and quinine and less susceptible to chloroquine than *P. falciparum* strain D6. Strain D6 is susceptible to pyrimethamine and sulphadoxine, but similar to *P. falciparum* strains TM90C2A and TM90C2B, and strain TM91C235 is less susceptible to mefloquine.

The effect of triclosan on *P. falciparum in vitro* was studied with pyrimethamine sensitive and resistant organisms, and those with varying sensitivity to chloroquine and mefloquine, simultaneously with studies of effect of chloroquin or mefloquine on these parasites (Table 1). Triclosan was effective against pyrimethamine resistant *P. falciparum* (W2) at low concentrations (IC50s of 150 nanograms/ml [triclosan] and 160 ngm/ml [Chloroquin], respectively) (Table 1). Interestingly, the pattern of relative susceptibility of triclosan and mefloquine were identical. This similarity suggests that triclosan and mefloquine may share a common mechanism of influx or efflux, because such differences in transporters are believed to be the basis of the differences in susceptibility of malaria parasites to mefloquin although other mechanisms are possible.

For *T. gondii,* growth inhibition was assessed over a 4-day period as described previously (Mack et al., 1984; Roberts et al., 1998; Zuther et al., 1999) using human foreskin fibroblasts (HFF) infected with 105 tachyzoites of the RH strain of *T. gondii.* The assays are based on microscopic visual inspection of infected and inhibitor treated cultures, and on quantitation of nucleic acid synthesis of the parasite by measuring uptake of 3H uracil into the parasite's nucleic acid. Uracil is not utilized by mammalian cells. Parasites present as tachyzoites (RH, Ptg., a clone derived from the Me49 strain), bradyzoites (Me49), and R5 mutants (mixed tachyzoites/bradyzoites of the Me49 strain that can be stage switched by culture conditions) (Bohne et al., 1993; Soete et al., 1994; Tomovo and Boothroyd, 1995; Weiss et al., 1992) are suitable for assay systems used to study effects of inhibitors. Only the RH strain tachyzoites, cultured for up to 72 hours, had been used in previously reported assays. The use of Me49, Ptg, and R5 mutants are unique aspects of the methods used in these assays in this invention.

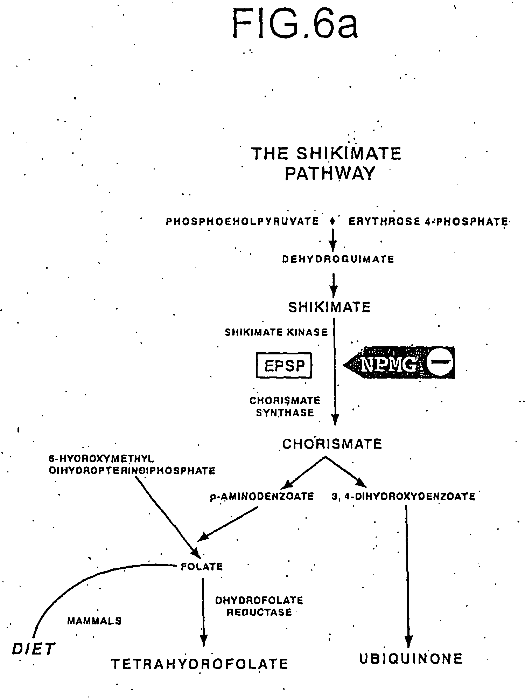

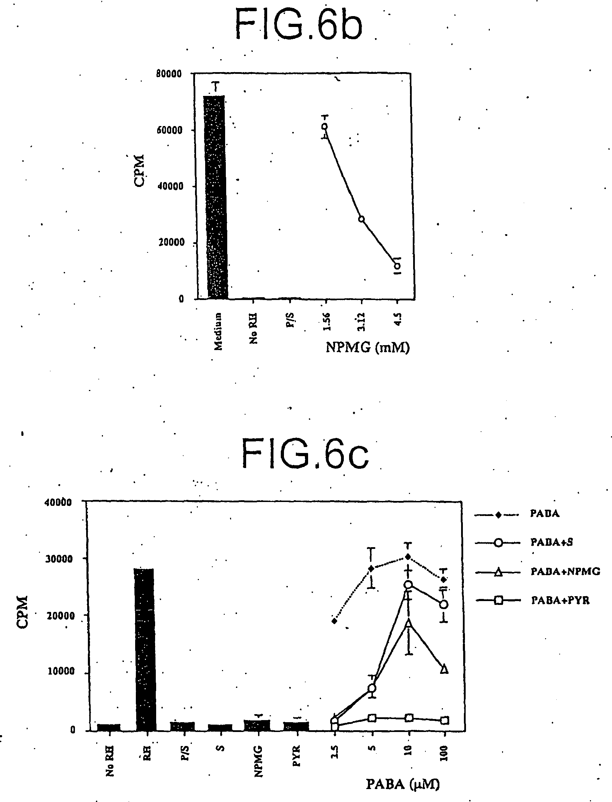

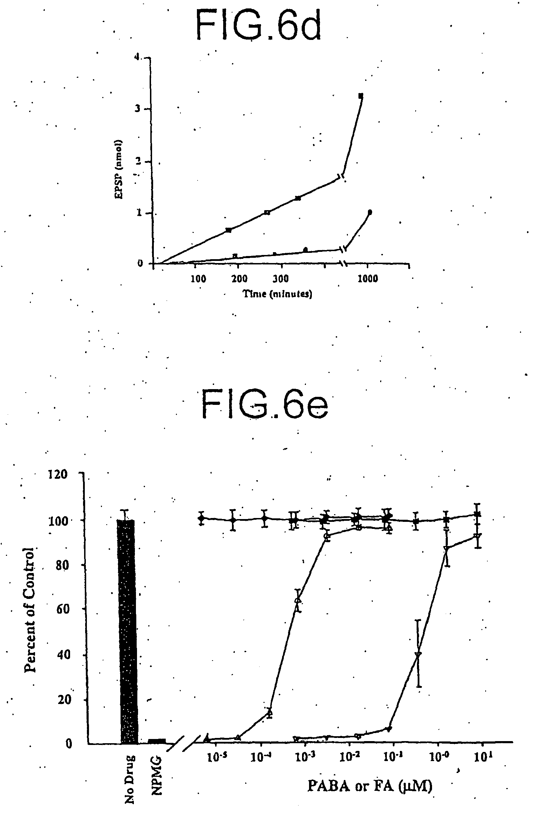

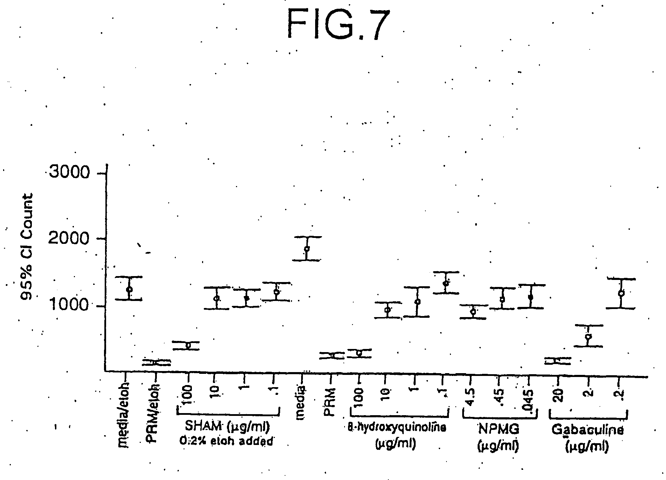

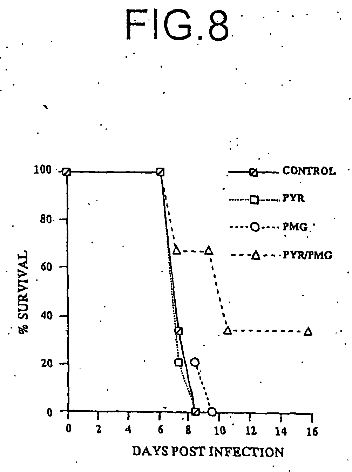

Results using the assay systems are shown in FIGS. 6-8. In these assays toxicity of a candidate inhibitor was assessed by its ability to prevent growth of human foreskin fibroblasts (HFF) after 4 days and after 8 days as measured by tritiated thymidine uptake and microscopic evaluation. Confluent monolayers of HFF were infected with tachyzoites and bradyzoites. Inhibitor was added one hour later. Non-toxic doses were used in parasite growth inhibition assays. Parasite growth was measured by ability to incorporate tritiated uracil during the last 48 hours of culture.

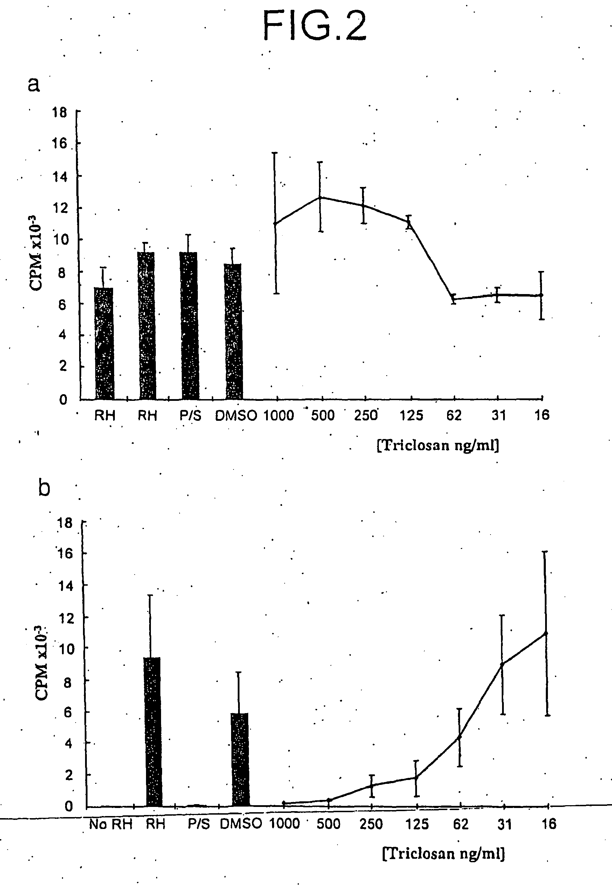

Triclosan also was effective against *T. gondii,* in nanomolar amounts (FIG. 2). IC50 was 62 nanograms/ml. There was no toxicity to host cells at these concentrations.

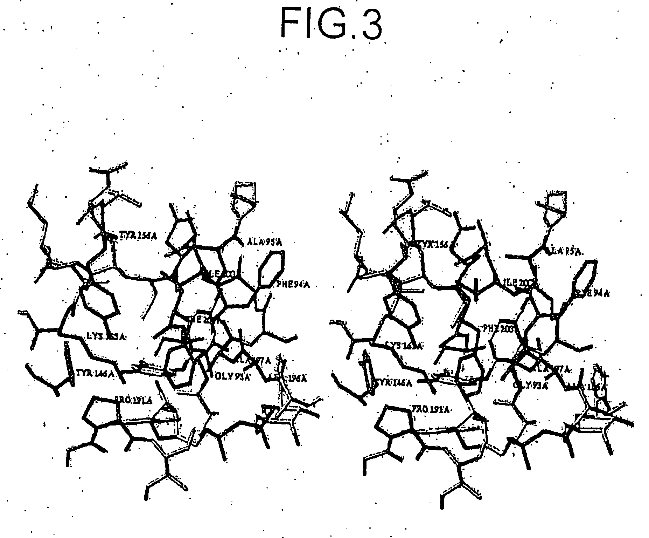

Analysis of the binding site for triclosan in *B. napus* and *E. coli* ENR shows that 11 residues have contacts less than 4Å with one of more atoms of the triclosan (FIG. 3). Inspection of the sequence for *P. falciparum* ENR reveals that it shares sequence identity at each of these positions with either the sequence of the *B. napus* or *E. coli* enzymes providing a clear explanation for the inhibitory properties of this agent against *P. falciparum*.

The discovery and characterization of an apicomplexan Fab I and discovery of triclosan as a lead compound provide means to rationally design novel inhibitory compounds with considerable promise. The invention provides novel ways to counteract the increasing resistance of Plasmodium to the current armoury of antimalarial agents and provides a new approach to the great need for additional, less toxic antimicrobial agents effective against *T. gondii.* The inventors (Zuther et al., 1999) and others (Waller et al., 1998) have also identified other novel inhibitors of sequential enzymatic steps in the apicomplexan lipid synthesis pathway, that are predicted to be synergistic with triclosan and other inhibitors of Fab I (Baldock, et al., 1996). This also raises the exciting possibility of a rational basis for discovery of synergistic inhibitors of this pathway effective against multiple different microorganisms (Payne et al., 2000).

### BRIEF DESCRIPTION OF THE DRAWINGS

FIG. 1 shows a multiple structure-based sequence alignment of the enoyl reductases from *E. coli, H. pylori, B. subtilis, S. aureus, P. falciparum* and *B. napus*. The secondary structures and sequence numbers of the *E. coli* and *B. napus* enzymes are shown above and below the alignment, respectively. The residues which are completely conserved are all black with white faced type and those involved in triclosan binding are indicated with a black, filled circle above. The N terminal sequence in the *P. falciparum* Fab I with no corresponding sequence in *E. coli* is a plastid target sequence which is a suitable separate target from the entire enzyme for inhibition.

FIG. 2 demonstrates inhibition of *T. gondii* by triclosan. (a) no inhibitory effect of triclosan on the host cells uptake of thymidine; appearance of the monolayer also was unchanged. (b) effect of triclosan on *T. gondii* uracil uptake; triclosan reduces uracil uptake by intracellular *T. gondii* 4 days after infection; IC50 was ≅ 62 nanograms per ml; effect increased between days 1 to 4, for example, in a separate experiment, for 125 nanograms per ml of triclosan on day 1, percentage inhibition was 20% and on day 4 was 72% and for pyrimethamine/sulfadiazine percentages of inhibition at these times were 63% and 100% respectively. Abbreviations: RH=RH strain of *T. gondii* within fibroblasts; No RH=control with fibroblasts alone; DMSO = fibroblasts with highest concentration of DMSO; P/S = fibroblasts, *T. gondii,* pyrimethamine and sulfadiazine used as a positive control for the assay; CPM = counts per minute.

FIG. 3 is a stereo view of the three dimensional arrangement of the atoms that form the binding pocket for triclosan, in *E. coli* enoyl reductase, with the 11 residues that have any atom within 4 A of the inhibitor, labeled. This is important in assigning the relative contributions made to the interaction with triclosan by the critical amino acids that are also present in the *P. falciparum* enzyme.

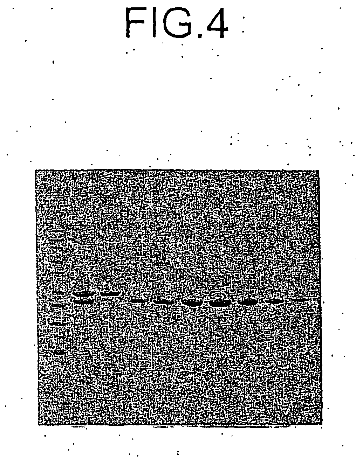

FIG. 4 shows *Fablt* fusion protein cut with Factor Xa protease (lane 2) was applied to a cation exchange column (SP Sepharose) separating the fusion protein (lane 3) from *Fab It* (lanes 4-10). Molecular weight markers (Sigma Wide) are shown in lane 1.

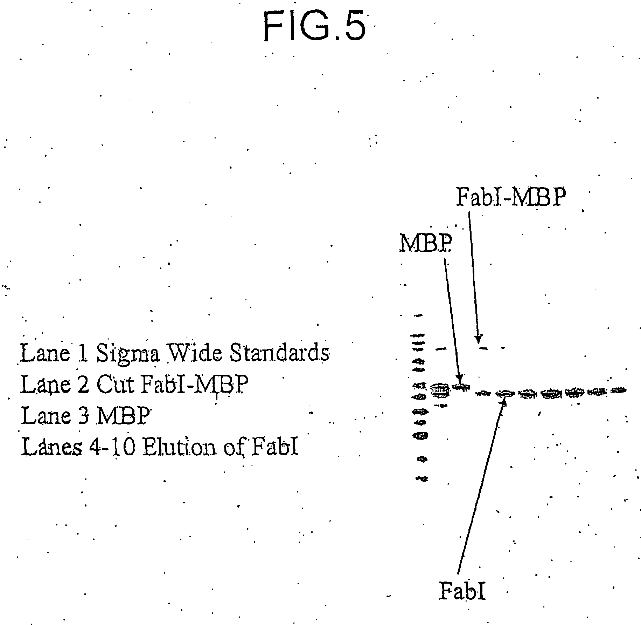

FIG. 5 shows the expression and purification of recombinant Fab I. FabI-MBP fusion protein cut with Factor Xa protease (lane 2) was applied to a cation exchange column (SP Sepharose) separating MBP (lane 3) from Fab I (lanes 4-10). A small amount of uncut fusion protein (FabI-MBP) can be seen in the elution fractions as well as some lower molecular weight fragments resulting from overdigestion of Fab I.

FIG. 6(A) is a schematic representation of the pathway for conversion of shikimate to chorismate in *T. gondii.* The inhibitor of EPSP synthase is NMPG; (B) shows uptake of tritiated uracil by tachyzoites (RH strain) is inhibited by NPMG. Toxicity of NPMG was assessed by its ability to prevent growth of human foreskin fibroblasts (HFF) after 4 days, as measured by tritiated thymidine uptake and microscopic evaluation; (C) shows product rescue of NPMG's inhibitory effect of EPSP synthase on PABA. The effect of PABA on sulfadiazine is similar, but the effect of pyrimethamine, as predicted reduces the enzyme to the levels that were present when media alone was utilized, as measured by the uracil uptake. S = sulfdiazine; PYR = pyrimethamine; and PABA = para amino benzoic acid; (D) shows functional and enzymatic evidence for the shikimate pathway in *T. gondii* with inhibition of EPSP synthase enzyme activity by 1mM glyosate. Squares, without glyphosate. Circles, with glyphosate; (E) shows evidence for the shikimate pathway in *P. falciparum* with functional evidence for the shikimate pathway in *P. falciparum.* Glyphosate inhibition of *in vitro* growth of asexual erythrocytic forms and PABA and folate antagonism of growth inhibition. Effect of NPMG on *C. parvum* was not abrogated by PABA. This suggests that either uptake of PABA by *C. parvum* differs or effect of NPMG is on a different branch from the shikimate pathway in *C. parvum*.

FIG. 7 shows inhibitory effects of NPMG, gabaculine, SHAM 8-OH-quinoline and on *Cryptosporidia*. 3NPA also inhibited *Cryptosporidia*.

FIG. 8 shows the effect of NPMG, pyrimethamine, and pyrimethamine plus NPMG on survival of mice following intraperitoneal infection with 500 tachyzoites of the RH strain of *T. gondii.* Dosage of NPMG was 200mg/kg/day and pyrimethamine was 12.5 mg/kg/day.

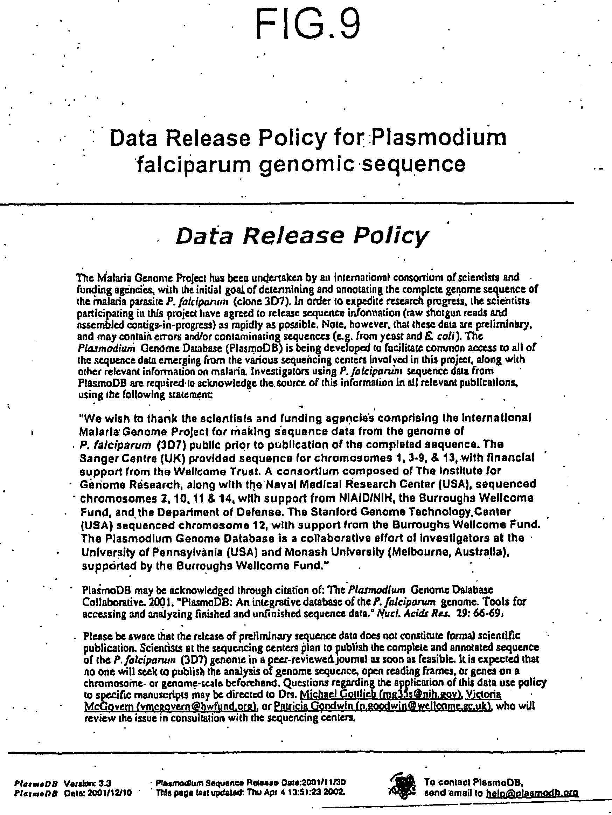

FIG. 9 is an illustrative copy of a web page for the *Plasmodium falciparum* genomic sequence.

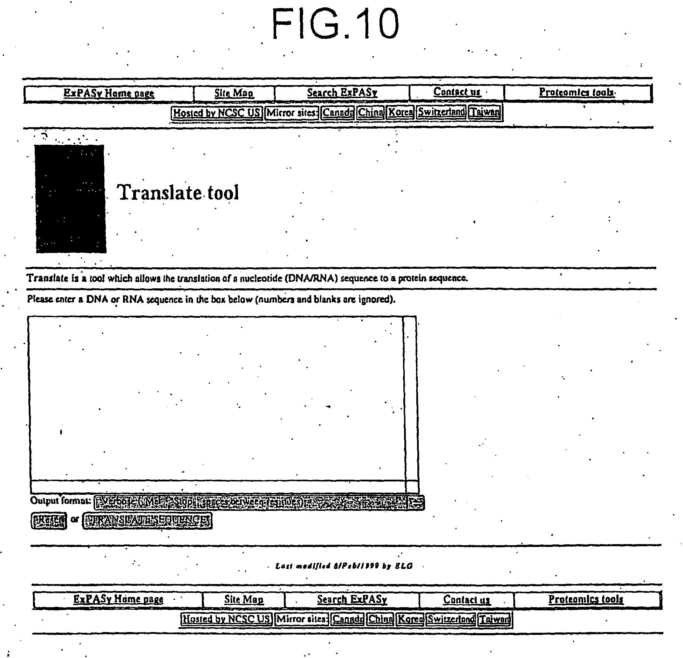

FIG. 10 is an illustrative copy of a web page with a tool to translate nucleotides to protein sequences.

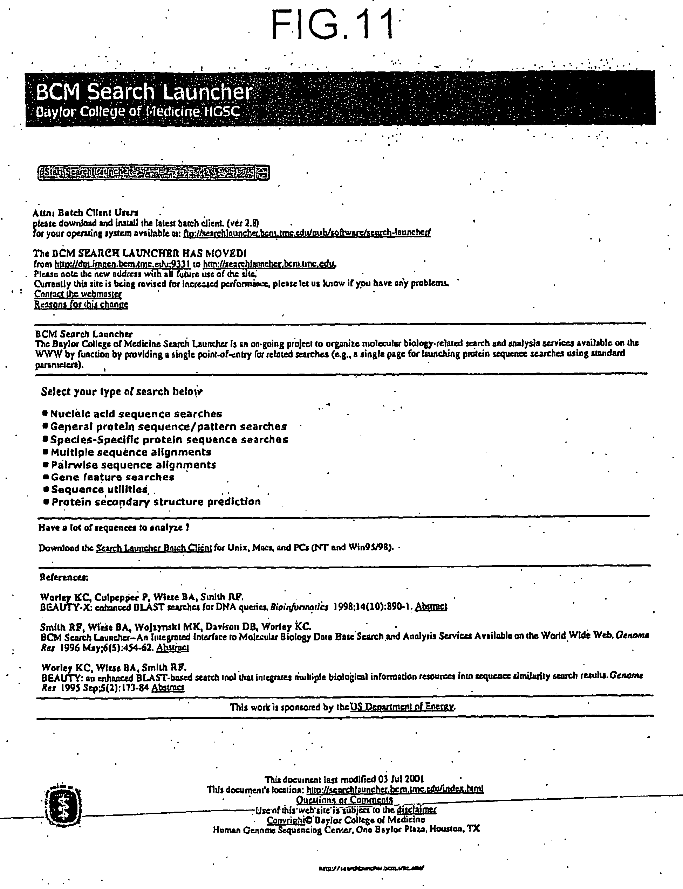

FIG. 11 is an illustrative copy of a web page for various searches.

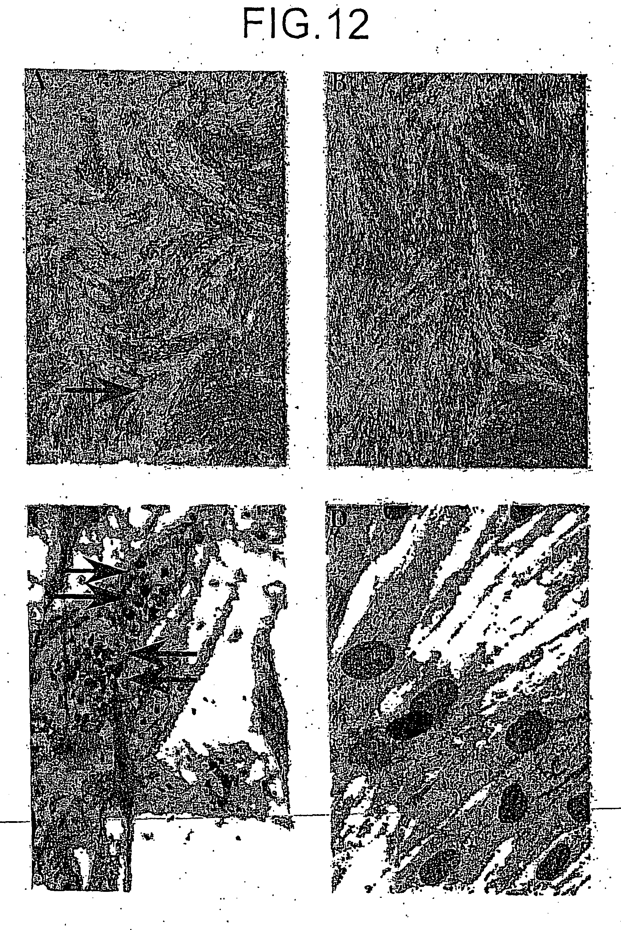

FIG. 12 shows acetyl Co-A carboxylases of apicomplexan were identified. *T. gondii* was ionhibited by the herbicide, clodinafop, 1 micromolar. A and C control; B and D with clodinafop.

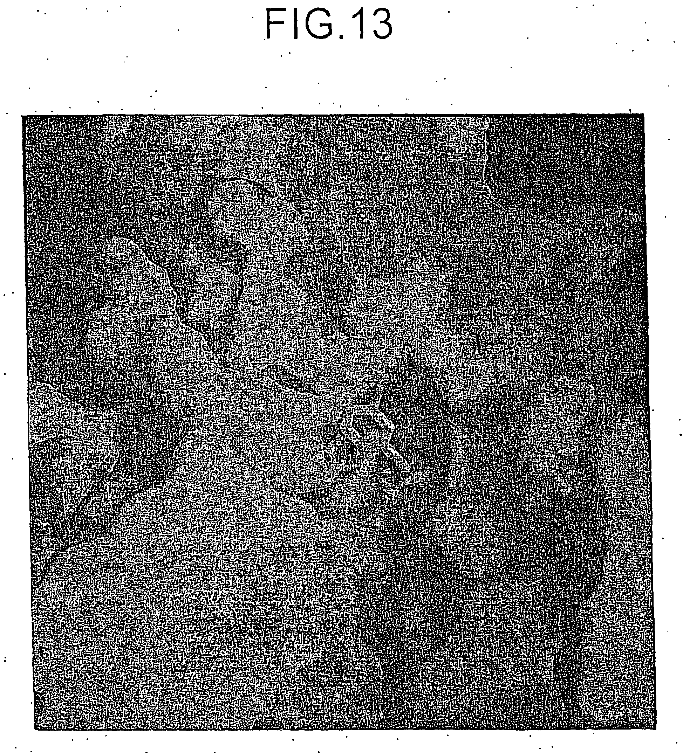

FIG. 13 shows a model of triclosan binding to its target enzyme, ENR.

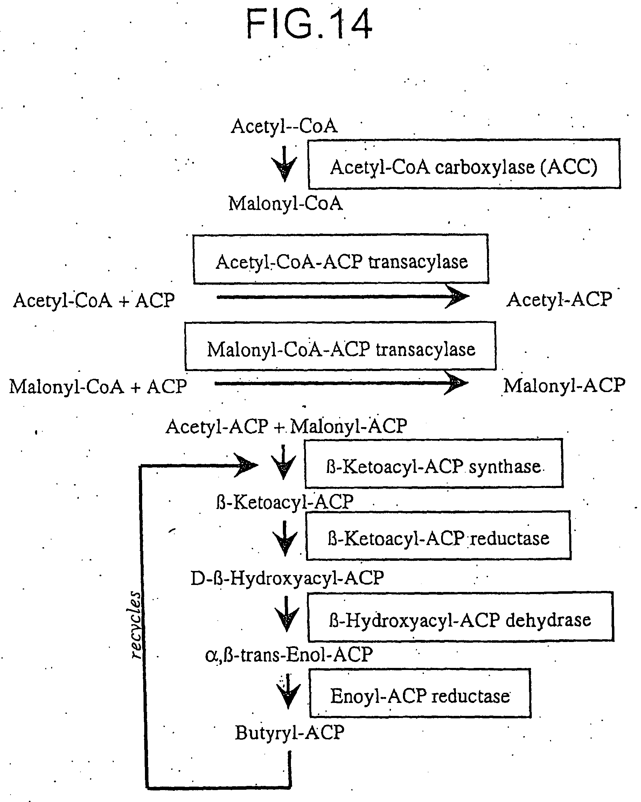

FIG. 14 shows the fatty acid synthesis pathway.

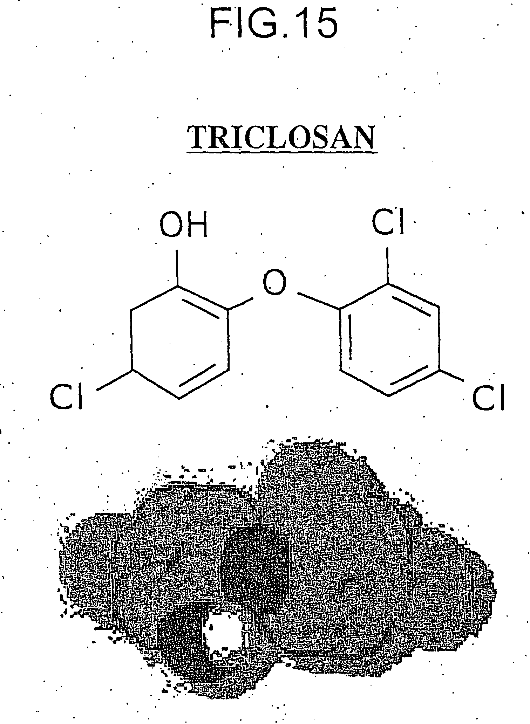

FIG. 15 is the molecular formula and model for triclosan.

### DETAILED DESCRIPTION OF THE INVENTION

A plant-like FAB I was identified in *Plasmodium falciparum.* The nucleotide sequence and deduced amino acid sequence was prepared and correct sequences were confirmed. FAB I is a single chain, discrete enzyme. All requisite residues for FAB I enzyme activity were confirmed. The *P. falciparum* enayl acyl carrier protein reductase has a putative plastid targeting sequence and unique polar insertions. The FAB I structure is modeled on *E. coli* and *B. napus* FAB I structure alone and complexed to triclosan. Key amino acids were identified for 2° structure. Residues for binding triclosan were conserved providing explanation for inhibition by triclosan. Triclosan inhibits *P. falciparum, T. gondii* (nm) in a pattern similar to the action of mefloquine. Soluble protein can be overexpressed.

Information obtained from *P. falciparum* because FAH I was purified include that the N terminal sequence is the same as *B. napus* FAB I, enzyme activity is NADH dependent and inhibited by triclosan. FAB I is involved in synthesis of 10, 12 C fatty acids. In a *P. berghei* murine model, Triclosan administered subcutaneously (3 or 38 mg/kg) was nontoxic, cleared parasitemia and prevented death. Synergy was demonstrated *in vitro* with cerulein, an inhibitor of Fab F, B, H.

### MATERIALS AND METHODS

*T. gondii in vitro*. Growth inhibition was assessed over a 4-day period as described previously by Roberts et al., 1998; Zuther et al., 1999; and Mack et al., 1984, all incorporated by reference, using human foreskin fibroblasts (HFF) infected with 105 tachyzoites of the RH strain of *T. gondii.* Uptake of 3H uracil was determined. Evaluation of slides of preparations containing HFF, Toxoplasma and inhibitors were made.

*P. falciparum*. The *in vitro* assays (Oduala et al., 1988; Milhous et al., 1985) were conducted using a modification of the semiautomated microdilution technique for assessing antifolate antagonists. Instead of dialysed human plasma, 10% Albumaz I (Gibco BRL), a serum-free substitute, was used to supplement the RPMI 16-40 media. All test compounds were dissolved in DMSO and diluted 400-fold into complete media before serial dilution over 11 concentrations. Incubation was at 37°C in 5% 02, 5% C 02 and 90% N2 for 48h, [3H]-Hypoxanthine incorporation was measured as described previously (13, 14). W2 is susceptible to mefloquine, but resistant to pyrimethamine, sulphadoxine, but similar to TM90C2A and TM90C2B, and TM91C235 is less susceptible to mefloquin.

**TABLE 1: IC 50¹ of TRICLOSAN, CHLOROQUINE, AND MEFLOQUINE WHEN CULTURED WITH P FALCIPARUM (NANOGRAMS/ML)**

| | Parasite Strain | | | | |
|---|---|---|---|---|---|
| Antimicrobial Agents | D6 | TM90C2A | W2 | TM90C2B | TM91C235 |
| Triclosan | 387.1 | 1891.4 | 154.4 | 1330.4 | 1800.5 |
| Mefloquine | 5.3 | 24.5 | 2.0 | 19.3 | 19.6 |
| Chloroquine | 3.8 | 57.3 | 162.4 | 82.7 | 46.1 |

| | | | | | |
|---|---|---|---|---|---|
| ¹ The activity of triclosan, mefloquine, and chloroquine were tested against a series of *P. falciparum* isolates and clones with differing susceptibilities to antimalarial drugs. D6, a clone from the African Sierra I/UNC isolate, is chloroquine and pyrimethamine susceptible; W2 is a clone of the Indochina I isolate and is chloroquine and pyrimethamine resistant. TM90C2A, TM90C2B, and TM91C235 are isolates from Thailand and all are chloroquine and mefloquine resistant. TM91C235 was isolated from a patient that failed mefloquine, twice, whereas TM90-C2a and TM90-Cb are admission and recrudescent isolates, respectively, of the first patient who failed treatment with atovaquone (alone) in Thailand. Subsequent susceptibility testing demonstrated that the recrudescent isolate (2B) was approximately 2000 fold resistant to atovaquone, when compared with the admission isolate and other atovaquone - susceptible isolates from Thailand. | | | | | |

**Cloning of the FabI gene**. The FabI gene from Plasmodium falciparum is located on chromosome 4 and codes for a 432 amino acid protein. The FabI gene from gDNA of the 3D7 strain of *P. falciparum* was amplified using PfuTurbo polymerase (Stratagene) and two primers (5'-GGTGGTGAATTCATGAATAAAATATCACAACGG-3' and 5'-GGTGGTGTCGACTTATTCATTTTCATTGCGATATATATC-3'). The resulting amplicon was digested with EcoRI and SalI endonucleases and gel purified using the QIAquick Gel Extraction Kit from Qiagen. The digested ampicon was ligated with T4 DNA ligase (Boehringer Mannheim) into the pMAL-c2x vector (New England Biolabs) which had previously been digested with the same endonucleases and treated with Shrimp Alkaline Phosphatase (USB). A second construct, lacking FabI residues 1-84, was prepared in the same way using the following two primers: 5'-GGTGGTGAATTCTCAAACATAAACAAAATTAAAGAAG-3' and 5'-GGTGGTGTCGACTTATTCATTTTCATTGCGATATATATC-3'. This truncated construct was called *FabIt.*

**Overexpression of *Fablt* in bacterial culture**. The pMAL-c2x vector containing the *Fablt* construct was transformed into BL21-CodonPlus(DE3) cells (Stratagene). Bacterial cultures were grown in shaker flasks at 37° to an OD600 of 0.6 and then induced with IPTG (Sigma) to a final concentration of 0.4mM. Induced cultures were transferred to a 20° shaker and incubated for an additional 12 hours. After this period, the cells were harvested by centrifugation at 5,000 x G for 15 minutes and the cell pellet was frozen at - 20°.

**Purification of Recombinant *Fab It* fusion protein**. Cell lysis buffer (20mM Na/K phosphate pH 7.5, 1 mg/ml lysozyme (Sigma), 2.5 µg/ml DNAse I (Sigma), 200mM NaCl) was added to the frozen cell pellets (20mL per liter of original culture) and gently vortexed. Resuspended cells were incubated on ice for 10 minutes followed by 30 seconds of sonication. Cell lysate was clarified by centrifugation at 20,000 x G for 15 minutes at 4° and applied to a 10ml amylose column (New England Biolabs) equilibrated in 20mM Na/K phosphate pH 7.5, 200mM NaCl. The column was washed with 5 column volumes of 20mM Na/K phosphate pH 7.5, 500mM NaCl followed by elution with 20mM Na/K phosphate pH 7.5, 200mM NaCl, 100mM Maltose.

**Cleavage of *FabIt* fusion protein and purification of *FabIt*.** Purified *Fablt* fusion protein was digested with Factor Xa (New England Biolabs) at ratio of 1 mg Factor Xa per 500mg of fusion protein. Calcium chloride was added to the reaction mixture at a final concentration of 1 mM and the mixture was incubated at 4° for 24 hours. The reaction mixture was desalted with a HiPrep 26/10 Desalting column (Pharmacia) equilibrated in 20mM Na/K phosphate pH 8.0, 10µM NAD+. Desalted protein was applied to a SP Sepharose cation exchange column (Pharmacia) equilibrated in 20mM Na/K phosphate pH 8.0, 10µM NAD+ and washed for 10 column volumes with the same buffer. Adsorbed proteins were eluted from the column with a linear gradient to 20mM Na/K phosphate pH 8.0, 10µM NAD+, 500mM NaCl in 20 column volumes. Fractions containing pure *Fablt* protein were pooled for further analysis.

**Overexpression of Recombinant Fab I**. The FabI gene was amplified from cDNA of the 3D7 strain of *P. falciparum* and inserted into the pMAL-c2x vector (New England Biolabs) for expression in *E. coli.* Recombinant FabI fused the Maltose Binding Protein (FabI-MBP) was purified from clarified cell lysate using a 10ml amylose column (New England Biolabs). The pure FabI-MBP fusion protein was cleaved with Factor Xa protease yielding FabI and MBP, which were the separated with a 5ml SP Sepharose column (Pharmacia).

### DOCUMENTS CITED

Baldock C., Rafferty J.B., Sdelnikova S.E., Baker P.J., Stitje A.R., Slabas A.R., Hawkes T.R., Rice D.W., 1996. A mechanism of drug action revealed by structural studies of enoyl reductase. Science. 274, 2107-2110.

DeRocher A., Hage C.B., Froehlich J.E., Feagin J.E., Parsons M., 2000. Analysis of targeting sequences demonstrates that trafficking to the toxoplasma gondii plastid branches off the secretory system. J Cell Sci. 113, (Pt22):3969-77.

Heath R.J., Yu Y.T., Shapiro M.A., Olson E. Rock CO., 1998. Broad-spectrum antimicrobial biocides target the Fab I component of fatty acid synthesis. J Biol Chem. 273,30316-30320.

Jomaa H., Wiesner J., Sanderbrand S., Altincicek B., Weidemeyer C., Hintz M., Turbachova I., Eberl M., Zeidler J., Lichtenhaler H.K., Soldati D., Beck E., 1999. Inhibitors of the nonmevalonate pathway of isoprenoid biosynthesis as antimalarial drugs. Science. 285 (5433):1573-6.

Jones R.D., Janpani H., Newman J.L., Lee A., 2000. Triclosan: A review of effectiveness and safety in healthcare settings. Am J. Inf. Control. 28, 184-196.

Levy, C.W., Roujeinikova A., Sedelnikova S.E., Baker P.J., Stuitje, A.R., Slabas, A.R., Rice, D.W., Rafferty, J.B., 1999. Molecular basis of triclosan activity. Nature. 398, 383-384 .

Mack D., McLeod R., 1984. A new micromethod to study effects of antimicrobial agents on Toxoplasma gondii: Comparison of sulfadoxine and sulfadiazine and study of clindamycin, metronidazole, and cyclosporin A. Antimicrob. Agents Chemother. 26, 26-30.

McMurray L.M., Oethinger M., Levy S.B., 1998. Triclosan targets lipid synthesis. Nature. 394, 531-2.

Milhous W.K., Weatherly N.F., Bowdre J.H., Desjardins R.E., 1985. In vitro activities and mechanisms of resistance to anti-folates and anti-malarial drugs. Antimicrob. Agents Chemother. 27,525-530.

Oduola A.M.J., Weatherly N.J., Bowdre J.H., Desjardins R.E., 1988. Plasmodium falciparum - cloning by single erythrocyte micromanipulation and heterogeneity in vitro. Exp. Parasitol. 66, 86-95.

Payne D.J., Wallis N.G., Gentry D.R., Rosenberg M., 2000. The impact of genomics on novel antibacterial targets. Curr Opin Drug Discovery Devel. 3(2), 177-190.

Plasmodium Genome Database Collaborative, 2001. PlasmoDB: An integrative database of the P. falciparum genome. Tools for accessing and analyzing finished and unfinshed sequence data. Nucl. Acids Res. 29: 66-69.

Roberts F., Roberts C.W., Johnson J., Kyle D.E., Krell T., Coggins J.R., Coombs G.H., Milhous W.K., Tzipori S., Ferguson D.J.P., Chakrabarti D., McLeod R., 1998. Evidence for the shikimate pathway in apicomplexan parasites. Nature. 393, 801-805.

Roos D.S., Crawford M.J., Donald R.G., Kissinger J.C., Klimczak L.J., Striepen B., 1999. Origin, targeting, and function of the apicomplexan plastid. Curr Opin Microbiol. 2(4):426-32. Review.

The Plasmodium Genome Database http://PlasmoDB.org (Nucleic Acids Research) 29, In Press 2001.

Turnowsky F. ,Fuchs K., Jeschek C., Hogenauer G., 1989. env M genes of Salmonella typhimurium and Escherichia coli. J. Bacteriol. 171, 6555-6565.

Waller R.F., Keeling P.J., Donald R.G., Striepen B., Handman E., Lan-Unnasch N., Cowman A.F., Besra G.S., Roos D.S., McFadden G.I., 1998. Nuclear-encoded proteins target to the plastid in Toxoplasma gondii and Plasmodium falciparum. Proc Natl Acad Sci USA. 95(21),12352-12357.

Waller R.F., Reed M.D., Cowman A.F., McFadden G.I., 2000. Protein trafficking to the plastid of Plasmodium falciparum is via the secretory pathway. EMBO J.19(8);1794-802.

Ward W.H.J., Holdgate G.A., Rowesell S., McLean E.G., Pauptit R.A., Clayton E., Nichols W.W., Colls J.G., Minshull C.A., Jude, D.A., Mistry A., Timms D., Camble R., Hales, N.J., Britton C.J., Taylor, I. W.F., 1999. Kinetic and structural characteristics of the inhibition of enoyl (acyl carrier protein) reductase by triclosan. Biochemistry. 38, 12514-12525.

Zuther E., Johnson J.J., Haselkorn R., McLeod R., and Gornicki P., 1999. Growth of Toxoplasma gondii is inhibited by aryloxyphenoxypropionate herbicides targeting acetyl-CoA carboxylase. Proc Natl. Acad Sci USA. 96, 13387-92.

HOANG TUNG T ET AL: "Characterization of Pseudomonas aeruginosa enoyl-acyl carrier protein reductase (FabI): A target for the antimicrobial triclosan and its role in acylated homoserine lactone synthesis" JOURNAL OF BACTERIOLOGY, WASHINGTON, DC, US, vol. 181, no. 17, 1999, pages 5489-5497, XP002194160 ISSN: 0021-9193

## Claims

1. A molecule of the Fab I enzyme having the amino acid sequence of the Fab I enzyme in *Plasmodium falciparum*, as shown in SEQ ID NO:5.

2. Use of the molecule according to claim 1 for developing inhibitors of *Plasmodium falciparum* or *Toxoplasma gondii in vitro*.

3. A nucleotide sequence encoding for the molecule according to claim 1.

## Patentansprüche

1. Molekül des Fab I Enzyms mit der Aminosäuresequenz des Fab I Enzyms in *Plasmodium falciparum*, wie in SEQ ID NO: 5 gezeigt.

2. Verwendung des Moleküls gemäß Anspruch 1 zum Entwickeln von Inhibitoren von *Plasmodium falciparum* oder *Toxoplasma gondii in vitro*.

3. Nukleotidsequenz, die für das Molekül gemäß Anspruch 1 kodiert.

## Revendications

1. Molécule de la enzyme Fab I avant la séquence d'acides aminés de la enzyme Fab I en *Plasmodium falciparum* présentée en SEQ ID NO: 5.

2. Utilisation de la molécule selon la revendication 1 pour le développement d'inhibiteurs de *Plasmodium falciparum* ou de *Toxoplasma gondii in vitro*.

3. Séquence de nucléotides codant la molécule selon la revendication 1.


### SEQUENCE LISTING

<110> MCLEOD, RIMA
   MUENCH, STEPHEN P.
   RAFFERTY, JOHN B.
   KYLE, DENNIS E.
   MUI, ERNEST J.
   KIRISITS, MICHAEL J.
   MACK, DOUGLAS G.
   ROBERTS, CRAIG W.
   SAMUEL, BENJAMIN U.
   LYONS, RUSSELL E.
   MILHOUS, WILBUR K.
   PRIGGE, SEAN
   RICE, DAVID W.
<120> FAB I AND INHIBITION OF APICOMPLEXAN PARASITES
<130> 223-28
<140> 10/465,527
   <141> 2003-06-18
<150> 01 986 546.8
   <151> 2001-12-20
<150> 60/257,771
   <151> 2000-12-21
<150> 60/264,499
   <151> 2001-01-26
<160> 10
<170> PatentIn Ver. 3.2
<210> 1
   <211> 262
   <212> PRT
   <213> Escherichia coli
<400> 1
   Met Gly Phe Leu Ser Gly Lys Arg Ile Leu Val Thr Gly Val Ala Ser
    1               5                  10                  15
   Lys Leu Ser Ile Ala Tyr Gly Ile Ala Gln Ala Met His Arg Glu Gly
               20                  25                  30
   Ala Glu Leu Ala Phe Thr Tyr Gln Asn Asp Lys Leu Lys Gly Arg Val
           35                  40                  45
   Glu Glu Phe Ala Ala Gln Leu Gly Ser Asp Ile Val Leu Gln Cys Asp
       50                  55                  60
   Val Ala Glu Asp Ala Ser Ile Asp Thr Met Phe Ala Glu Leu Gly Lys
   65                  70                  75                  80
   Val Trp Pro Lys Phe Asp Gly Phe Val His Ser Ile Gly Phe Ala Pro
                   85                  90                  95
   Gly Asp Gln Leu Asp Gly Asp Tyr Val Asn Ala Val Thr Arg Glu Gly
               100                 105                 110
   Phe Lys Ile Ala His Asp Ile Ser Ser Tyr Ser Phe Val Ala Met Ala
   115 120 125
   Lys Ala Cys Arg Ser Met Leu Asn Pro Gly Ser Ala Leu Leu Thr Leu
   130 135 140
   Ser Tyr Leu Gly Ala Glu Arg Ala Ile Pro Asn Tyr Asn Val Met Gly
   145 150 155 160
   Leu Ala Lys Ala Ser Leu Glu Ala Asn Val Arg Tyr Met Ala Asn Ala
   165 170 175
   Met Gly Pro Glu Gly Val Arg Val Asn Ala Ile Ser Ala Gly Pro Ile
   180 185 190
   Arg Thr Leu Ala Ala Ser Gly Ile Lys Asp Phe Arg Lys Met Leu Ala
   195 200 205
   His Cys Glu Ala Val Thr Pro Ile Arg Arg Thr Val Thr Ile Glu Asp
   210 215 220
   Val Gly Asn Ser Ala Ala Phe Leu Cys Ser Asp Leu Ser Ala Gly Ile
   225 230 235 240
   Ser Gly Glu Val Val His Val Asp Gly Gly Phe Ser Ile Ala Ala Met
   245 250 255
   Asn Glu Leu Glu Leu Lys
   260
<210> 2
   <211> 275
   <212> PRT
   <213> Helicobacter pylori
<400> 2
   Met Gly Phe Leu Lys Gly Lys Lys Gly Leu Ile Val Gly Val Ala Asn
   1               5                   10                  15
   Asn Lys Ser Ile Ala Tyr Gly Ile Ala Gln Ser Cys Phe Asn Gln Gly
               20                  25                  30
   Ala Thr Leu Ala Phe Thr Tyr Leu Asn Glu Ser Leu Glu Lys Arg Val
           35                  40                  45
   Arg Pro Ile Ala Gln Glu Leu Asn Ser Pro Tyr Val Tyr Glu Leu Asp
       50                  55                  60
   Val Ser Lys Glu Glu His Phe Lys Ser Leu Tyr Asn Ser Val Lys Lys
   65                  70                  75                  80
   Asp Leu Gly Ser Leu Asp Phe Ile Val His Ser Val Ala Phe Ala Pro
                   85                  90                  95
   Lys Glu Ala Leu Glu Gly Ser Leu Leu Glu Thr Ser Lys Ser Ala Phe
               100                 105                 110
   Asn Thr Ala Met Glu Ile Ser Val Tyr Ser Leu Ile Glu Leu Thr Asn
           115                 120                 125
   Thr Leu Lys Pro Leu Leu Asn Asn Gly Ala Ser Val Leu Thr Leu Ser
       130                 135                 140
   Tyr Leu Gly Ser Thr Lys Tyr Met Ala His Tyr Asn Val Met Gly Leu
   145                 150                 155                 160
   Ala Lys Ala Ala Leu Glu Ser Ala Val Arg Tyr Leu Ala Val Asp Leu
                   165                 170                 175
   Gly Lys His His Ile Arg Val Asn Ala Leu Ser Ala Gly Pro Ile Arg
               180                 185                 190
   Thr Leu Ala Ser Ser Gly Ile Ala Asp Phe Arg Met Ile Leu Lys Trp
           195                 200                 205
   Asn Glu Ile Asn Ala Pro Leu Arg Lys Asn Val Ser Leu Glu Glu Val
       210                 215                 220
   Gly Asn Ala Gly Met Tyr Leu Leu Ser Ser Leu Ser Ser Gly Val Ser
   225                 230                 235                 240
   Gly Glu Val His Phe Val Asp Ala Gly Tyr His Val Met Gly Met Gly
                   245                 250                 255
   Ala Val Glu Glu Lys Asp Asn Lys Ala Thr Leu Leu Trp Asp Leu His
               260                 265                 270
   Lys Glu Gln
           275
<210> 3
   <211> 258
   <212> PRT
   <213> Bacillus subtilis
<400> 3
   Met Asn Phe Ser Leu Glu Gly Arg Asn Ile Val Val Met Gly Val Ala
   1               5                   10                  15
   Asn Lys Arg Ser Ile Ala Trp Gly Ile Ala Arg Ser Leu His Glu Ala
               20                  25                  30
   Gly Ala Arg Leu Ile Phe Thr Tyr Ala Gly Glu Arg Leu Glu Lys Ser
           35                  40                  45
   Val His Glu Leu Ala Gly Thr Leu Asp Arg Asn Asp Ser Ile Ile Leu
       50                  55                  60
   Pro Cys Asp Val Thr Asn Asp Ala Glu Ile Glu Thr Cys Phe Ala Ser
   65                  70                  75                  80
   Ile Lys Glu Gln Val Gly Val Ile His Gly Ile Ala His Cys Ile Ala
                   85                  90                  95
   Phe Ala Asn Lys Glu Glu Leu Val Gly Glu Tyr Leu Asn Thr Asn Arg
               100                 105                 110
   Asp Gly Phe Leu Leu Ala His Asn Ile Ser Ser Tyr Ser Leu Thr Ala
           115                 120                 125
   Val Val Lys Ala Ala Arg Pro Met Met Thr Glu Gly Gly Ser Ile Val
       130                 135                 140
   Thr Leu Thr Tyr Leu Gly Gly Glu Leu Val Met Pro Asn Tyr Asn Val
   145                 150                 155                 160
   Met Gly Val Ala Lys Ala Ser Leu Asp Ala Ser Val Lys Tyr Leu Ala
                   165                 170                 175
   Ala Asp Leu Gly Lys Glu Asn Ile Arg Val Asn Ser Ile Ser Ala Gly
               180                 185                 190
   Pro Ile Arg Thr Leu Ser Ala Lys Gly Ile Ser Asp Phe Asn Ser Ile
           195                 200                 205
   Leu Lys Asp Ile Glu Glu Arg Ala Pro Leu Arg Arg Thr Thr Thr Pro
       210                 215                 220
   Glu Glu Val Gly Asp Thr Ala Ala Phe Leu Phe Ser Asp Met Ser Arg
   225                 230                 235                 240
   Gly Ile Thr Gly Glu Asn Leu His Val Asp Ser Gly Phe His Ile Thr
                   245                 250                 255
   Ala Arg
<210> 4
   <211> 264
   <212> PRT
   <213> Staphylococcus aureus
<400> 4
   Met Thr Thr Lys Ile Ser Met Leu Asn Leu Thr Gly Lys Asn Ala Leu
   1               5                   10                  15
   Val Thr Gly Ile Ala Asn Asn Arg Ser Ile Ala Trp Gly Ile Ala Gln
               20                  25                  30
   Gln Leu His Ala Ala Gly Ala Asn Leu Gly Ile Thr Tyr Leu Pro Asp
           35                  40                  45
   Glu Arg Gly Lys Phe Glu Lys Lys Val Ser Glu Leu Val Glu Pro Leu
       50                  55                  60
   Asn Pro Ser Leu Phe Leu Pro Cys Asn Val Gln Asn Asp Glu Gln Ile
   65                  70                  75                  80
   Gln Ser Thr Phe Asp Thr Ile Arg Asp Lys Trp Gly Arg Leu Asp Ile
                   85                  90                  95
   Leu Ile His Cys Leu Ala Phe Ala Asn Arg Asp Asp Leu Thr Gly Asp
               100                 105                 110
   Phe Ser Gln Thr Ser Arg Ala Gly Phe Ala Thr Ala Leu Asp Ile Ser
           115                 120                 125
   Thr Phe Ser Leu Val Gln Leu Ser Gly Ala Ala Lys Pro Leu Met Thr
       130                 135                 140
   Glu Gly Gly Ser Ile Ile Thr Leu Ser Tyr Leu Gly Gly Val Arg Ala
   145                 150                 155                 160
   Val Pro Asn Tyr Asn Val Met Gly Val Ala Lys Ala Gly Leu Glu Ala
                   165                 170                 175
   Ser Val Arg Tyr Leu Ala Ser Glu Leu Gly Ser Gln Asn Ile Arg Val
               180                 185                 190
   Asn Ala Ile Ser Ala Gly Pro Ile Arg Thr Leu Ala Ser Ser Ala Val
           195                 200                 205
   Gly Gly Ile Leu Asp Met Ile His His Val Glu Gln Val Ala Pro Leu
       210                 215                 220
   Arg Arg Thr Val Thr Gln Leu Glu Val Gly Asn Thr Ala Ala Phe Leu
   225                 230                 235                 240
   Ala Ser Asp Leu Ala Ser Gly Ile Thr Gly Gln Val Leu Tyr Val Asp
                   245                 250                 255
   Ala Gly Tyr Glu Ile Met Gly Met
               260
<210> 5
   <211> 420
   <212> PRT
   <213> Plasmodium falciparum
<400> 5
   Met Asn Lys Ile Ser Gln Arg Leu Leu Phe Leu Phe Leu His Phe Tyr
    1               5                  10                  15
   Thr Ile Val Cys Phe Ile Gln Asn Asn Thr Gln Lys Thr Phe His Asn
               20                  25                  30
   Val Leu His Asn Glu Gln Ile Arg Gly Lys Glu Lys Ala Phe Tyr Arg
           35                  40                  45
   Lys Glu Lys Arg Glu Asn Ile Phe Ile Gly Asn Lys Met Lys His Leu
       50                  55                  60
   Asn Asn Met Asn Asn Thr His Asn Asn Asn His Tyr Met Glu Lys Glu
   65                  70                  75                  80
   Glu Gln Asp Ala Ser Asn Ile Tyr Lys Ile Lys Glu Glu Asn Lys Asn
                   85                  90                  95
   Glu Asp Ile Cys Phe Ile Ala Ile Gly Asp Thr Asn Gly Tyr Gly Trp
               100                 105                 110
   Gly Ile Lys Glu Leu Ser Lys Arg Asn Val Lys Ile Ile Phe Gly Ile
           115                 120                 125
   Trp Pro Pro Val Tyr Asn Ile Phe Met Lys Asn Tyr Lys Asn Gly Lys
       130                 135                 140
   Phe Asp Asn Asp Met Ile Ile Asp Lys Asp Lys Lys Met Asn Ile Leu
   145                 150                 155                 160
   Asp Met Leu Pro Phe Asp Ala Ser Phe Asp Thr Ala Asn Asp Ile Asp
                   165                 170                 175
   Glu Glu Thr Lys Asn Asn Lys Arg Tyr Asn Met Leu Gln Asn Tyr Thr
               180                 185                 190
   Ile Glu Asp Val Ala Asn Leu Ile His Gln Lys Tyr Gly Lys Ile Asn
           195                 200                 205
   Met Leu Val His Ser Leu Ala Asn Ala Lys Glu Val Gln Lys Lys Asp
       210                 215                 220
   Leu Leu Asn Thr Ser Arg Lys Gly Tyr Leu Asp Leu Ser Lys Ser Tyr
   225                 230                 235                 240
   Leu Ile Ser Leu Cys Lys Tyr Phe Val Asn Ile Met Lys Pro Gln Ser
                   245                 250                 255
   Ser Ile Ile Ser Thr His Ala Ser Gln Lys Val Val Pro Gly Gly Gly
               260                 265                 270
   Gly Ser Ser Ala Leu Glu Ser Asp Thr Arg Val Ala Tyr His Leu Gly
           275                 280                 285
   Arg Asn Tyr Asn Ile Arg Ile Asn Thr Ile Ser Ala Gly Pro Leu Lys
       290                 295                 300
   Ser Arg Ala Ala Thr Ala Ile Asn Lys Leu Asn Asn Thr Tyr Glu Asn
   305                 310                 315                 320
   Asn Thr Asn Gln Asn Lys Asn Arg Asn Ser His Asp Val His Asn Ile
                   325                 330                 335
   Met Asn Asn Ser Gly Glu Lys Glu Glu Lys Lys Asn Ser Ala Ser Gln
               340                 345                 350
   Asn Tyr Thr Phe Ile Asp Tyr Ala Ile Glu Tyr Ser Glu Lys Tyr Ala
           355                 360                 365
   Pro Leu Arg Gln Lys Leu Leu Ser Thr Asp Ile Gly Ser Val Ala Ser
       370                 375                 380
   Phe Leu Leu Ser Arg Glu Ser Arg Ala Ile Thr Gly Gln Thr Ile Tyr
   385                 390                 395                 400
   Val Asp Asn Gly Leu Asn Ile Met Phe Leu Pro Asp Asp Ile Tyr Arg
                   405                 410                 415
   Asn Glu Asn Glu
               420
<210> 6
   <211> 374
   <212> PRT
   <213> Brassica napus
<400> 6
   Met Ala Ala Thr Ala Ala Ala Ser Ser Leu Gln Met Ala Thr Thr Arg
    1               5                  10                  15
   Pro Ser Ile Ser Ala Ala Ser Ser Lys Ala Arg Thr Tyr Val Val Gly
               20                  25                  30
   Ala Asn Pro Arg Asn Ala Tyr Lys Ile Ala Cys Thr Pro His Leu Ser
           35                  40                  45
   Asn Leu Gly Cys Leu Arg Asn Asp Ser Ala Leu Pro Ala Ser Lys Lys
       50                  55                  60
   Ser Phe Ser Phe Ser Thr Lys Ala Met Ser Glu Ser Ser Glu Ser Lys
   65                  70                  75                  80
   Ala Ser Ser Gly Leu Pro Ile Asp Leu Arg Gly Lys Arg Ala Phe Ile
                    85                  90                  95
   Ala Ile Ala Asp Asp Asn Gly Tyr Gly Trp Ala Val Lys Ser Leu Ala
               100                 105                 110
   Ala Ala Gly Ala Glu Ile Leu Val Gly Thr Trp Val Pro Ala Leu Asn
           115                 120                 125
   Ile Phe Glu Thr Ser Leu Arg Arg Gly Lys Phe Asp Gln Ser Arg Val
       130                 135                 140
   Leu Pro Asp Gly Ser Leu Met Glu Ile Lys Lys Val Tyr Pro Leu Asp
   145                 150                 155                 160
   Ala Val Phe Asp Asn Pro Glu Asp Val Pro Glu Asp Val Lys Ala Asn
                   165                 170                 175
   Lys Arg Tyr Ala Gly Ser Ser Asn Trp Thr Val Gln Glu Ala Ala Glu
               180                 185                 190
   Cys Val Arg Gln Asp Phe Gly Ser Ile Asp Ile Leu Val His Ser Leu
           195                 200                 205
   Ala Asn Gly Pro Glu Val Ser Lys Lys Pro Leu Leu Glu Thr Ser Arg
       210                 215                 220
   Lys Gly Tyr Leu Ala Ile Ser Ala Ser Tyr Phe Val Ser Leu Leu Ser
   225                 230                 235                 240
   His Phe Leu Pro Ile Met Asn Pro Gly Gly Ala Ser Ile Ser Thr Ile
                   245                 250                 255
   Ala Ser Glu Arg Ile Ile Pro Gly Gly Gly Gly Ser Ser Ala Leu Glu
               260                 265                 270
   Ser Asp Thr Arg Val Leu Ala Phe Glu Ala Gly Arg Lys Gln Asn Ile
           275                 280                 285
   Arg Val Asn Thr Ile Ser Ala Gly Pro Leu Gly Ser Arg Ala Ala Lys
       290                 295                 300
   Ala Ile Gly Phe Ile Asp Thr Met Ile Glu Tyr Ser Tyr Asn Asn Ala
   305                 310                 315                 320
   Pro Ile Gln Lys Thr Leu Thr Ala Asp Glu Val Gly Asn Ala Ala Ala
                   325                 330                 335
   Phe Leu Val Ser Pro Leu Ala Ser Ala Ile Thr Gly Ala Thr Ile Tyr
               340                 345                 350
   Val Asp Asn Gly Leu Asn Ser Met Gly Val Ala Leu Asp Ser Pro Val
           355                 360                 365
   Phe Lys Asp Leu Asn Lys
       370
<210> 7
   <211> 33
   <212> DNA
   <213> Artificial Sequence
<220>
   <223> Description of Artificial Sequence: Synthetic primer
<400> 7
   ggtggtgaat tcatgaataa aatatcacaa egg 33
<210> 8
   <211> 39
   <212> DNA
   <213> Artificial Sequence
<220>
   <223> Description of Artificial Sequence: Synthetic primer
<400> 8
   ggtggtgtcg acttattcat tttcattgcg atatatatc 39
<210> 9
   <211> 37
   <212> DNA
   <213> Artificial Sequence
<220>
   <223> Description of Artificial Sequence: Synthetic primer
<400> 9
   ggtggtgaat tctcaaacat aaacaaaatt aaagaag 37
<210> 10
   <211> 39
   <212> DNA
   <213> Artificial Sequence
<220>
   <223> Description of Artificial Sequence: Synthetic primer
<400> 10
   ggtggtgtcg acttattcat tttcattgcg atatatatc 39